**Europäisches Patentamt**

(19) **European Patent Office**

**Office européen des brevets**

(11) Veröffentlichungsnummer : **0 158 120 B2**

(12) **NEUE EUROPÄISCHE PATENTSCHRIFT**

(45) Veröffentlichungstag der neuen Patentschrift :
**10.06.92 Patentblatt 92/24**

(51) Int. Cl.[5] : **A61K 9/14,** A23K 1/16,
A23K 1/175

(21) Anmeldenummer : **85102542.9**

(22) Anmeldetag : **06.03.85**

(54) Verfahren zur Herstellung von fliessfähigen Cholinchlorid-Kieselsäure-Pulvern.

Die Akte enthält technische Angaben, die nach dem Eingang der Anmeldung eingereicht wurden und die nicht in dieserPatentschrift enthalten sind.

(30) Priorität : **13.03.84 DE 3409063**

(43) Veröffentlichungstag der Anmeldung :
**16.10.85 Patentblatt 85/42**

(45) Bekanntmachung des Hinweises auf die Patenterteilung :
**25.05.88 Patentblatt 88/21**

(45) Bekanntmachung des Hinweises auf die Entscheidung über den Einspruch :
**10.06.92 Patentblatt 92/24**

(84) Benannte Vertragsstaaten :
**BE DE FR GB NL**

(56) Entgegenhaltungen :
**EP-A- 0 074 050
DD-A- 84 552
DE-A- 1 619 865
DE-A- 2 209 477
DE-B- 1 692 417
FR-A- 2 518 426
US-A- 2 879 161**

(56) Entgegenhaltungen :
**CHEMICAL ABSTRACTS, Band 81, Nr. 5, 5. August 1974, Seite 384, Nr. 25051c, Columbus, Ohio, US
Broschüre Huber 1973. Zeosyl (R) 110 SD
Broschüre ICI PC/PD/122E/874/258 "Choline chloride"
Bulletin Technique Pigments, Degussa no. 50 "Sipernat" 1970
Broschüre Rhone-Poulenc Chimie de Base "Tix-O-Lex 28, Tix-O-Sil 38-38a" 20.05.80**

(73) Patentinhaber : **BASF Aktiengesellschaft
Carl-Bosch-Strasse 38
W-6700 Ludwigshafen (DE)**

(72) Erfinder : **Hertel, Otto, Dr.
Koenigsbacher Strasse 68
W-6700 Ludwigshafen (DE)**
Erfinder : **Jeschek, Gerhard, Dr.
Im Zaunruecken 14
W-6718 Gruenstadt (DE)**
Erfinder : **Klink, Walter, Dr.
Schlesier Strasse 11
W-6701 Birkenheide (DE)**
Erfinder : **Koernig, Wolfgang, Dr.
Dachsweg 5
W-6901 Dossenheim (DE)**
Erfinder : **Weber, Theodor
Virchowstrasse 20
W-6700 Ludwigshafen (DE)**
Erfinder : **Rateike, Fritz
Wormser Strasse 74 a
W-6840 Lampertheim 1 (DE)**

EP 0 158 120 B2

**Beschreibung**

Die vorliegende Erfindung betrifft ein neues Verfahren zur Herstellung von fließfähigen Cholinchlorid-Kieselsäure-Pulvern durch Mischen von Kieselsäure mit einer wäßrigen Cholinchloridlösung und Zugabe von Magnesiumstearat oder Calciumstearat.

Cholinchlorid ist ein wichtiger Bestandteil von Tierfuttermischungen. Dazu wird es überwiegend in pulverförmigem Zustand eingesetzt. Man verwendet dabei Cholinchlorid in Substanz oder aufgebracht auf ein Trägermaterial. Als Trägermaterialien dienen Kieselsäure oder organische Trägerstoffe, beispielsweise Maisspindelmehl oder Reisschalen.

In den auf Träger aufgebrachten Cholinchlorid-Pulvern beträgt die Konzentration an Cholinchlorid im allgemeinen ca. 50 Gew.%; aber auch Pulver mit höheren oder geringeren Konzentrationswerten an Cholinchlorid sind bekannt und handelsüblich.

Produkte mit einem Träger aus Kieselsäure sind überwiegend Adsorbate und weisen in der Regel eine Restwassermenge zwischen 15 und 25 Gew.% auf. Produkte mit organischen Trägerstoffen sind üblicherweise getrocknet und besitzen Restwassermengen von ca. 1 bis 2 Gew.%.

Da Cholinchlorid hygroskopisch ist, neigen die genannten Cholinchlorid-Pulver zum Verbacken und werden dadurch schlecht rieselfähig, wenn sie mit Feuchtigkeit, z. B. mit Luftfeuchtigkeit, in Berühung kommen. Auch frisch hergestellte Produkte haben aufgrund von Restwassermengen an der Oberfläche meist keine gute Rieselfähigkeit.

Eine gute Rieselfähigkeit ist aber für die Weiterverarbeitung zu Futtermitteln, speziell bei Praemixen, unbedingt erforderlich. Es muß zudem gewährleistet sein, daß auch bei mehrstündigem Lagern von unverschlossenen Gebinden, die Cholinchlorid-Pulver enthalten, die Neigung zum Verbacken dieser Pulver möglichst gering ist und somit eine gute Rieselfähigkeit erhalten bleibt.

Es ist bekannt, daß sich die unerwünschte Verbackung von trägerfreien Cholinchlorid-Pulvern durch Zusatz größerer Mengen von Salzen der Stearinsäure zurückdrängen läßt.

Nach dem Vorschlag der NL-A-6 704 009 soll bei der Herstellung von trägerfreiem Cholinchlorid-Pulver Natriumstearat in einem Anteil von ca. 20 Gew.%, bezogen auf die Mischung, zugesetzt werden.

In der DD-A-84 552 wird beschrieben, trägerfreies Cholinchlorid mit Magnesiumstearat oder Calciumstearat zu stabilisieren, wobei der Anteil der Stearate in den Ausführungsbeispielen bei 2,9 bis 4,8 Gew.% bezogen auf die Mischung, liegt.

In der Firmenschrift "Zeosyl 110 SD" (Huber) werden fließfähige Cholinchlorid-Pulver mit Kieselsäure (Zeosyl 110 SD) als Trägermaterial beschrieben, die zur Verbesserung der Fließfähigkeit und der Lagerstabilität 0,5 bzw. 3 Gew.-% Magnesiumstearat enthalten können. Die Herstellung erfolgt durch Mischen einer 75-80 gew.-%igen Cholinchlorid-Lösung mit etwa der halben Menge Zeosyl 110 SD (bezogen auf das Gewicht der Lösung) bei Raumtemperatur. Nachteilig an diesen Mischungen ist, daß mit steigendem Cholinchlorid-Gehalt die Konzentration an Magnesiumstearat erhöht werden muß.

Aufgabe der vorliegenden Erfindung war es nun, ein Verfahren zur Herstellung von fließfähigen und nicht zum Verbacken neigenden Cholinchlorid-Pulvern, die einen Kieselsäureträger besitzen, bereitzustellen, wobei gleichzeitig der Zusatz von größeren Mengen an teueren Stearinsäuresalzen vermieden werden sollte.

Es wurde gefunden, daß man fließfähige Cholinchlorid-Kieselsäure-Pulver durch Mischen von Kieselsäure mit Cholinchlorid, Zugeben von einem Erdalkalistearat und gegebenenfalls Trocknung dieser Mischung vorteilhaft erhält, wenn man sprüh- oder wirbelschichtgetrocknete Kieselsäure mit einer Korngröße von 30 bis 250 µm, die zu mehr als 50 Gew.% eine Korngröße von 50 bis 160 µm aufweist, mit einer wäßrigen Cholinchloridlösung, die 70 bis 80 Gew.%, bezogen auf die Lösung an Cholinchlorid enthält und deren Temperatur 40 bis 80 °C beträgt, mischt und anschließend 0,05 bis 0,4 Gew.%, bezogen auf die Mischung, an Magnesiumstearat oder Calciumstearat zugibt und diese Mischung gegebenenfalls trocknet.

Dies ist überraschend, da, wie bereits erwähnt, bei trägerfreiem Cholinchlorid eine Verbesserung der Fließfähigkeit nur bei Zugabe größerer Mengen an Stearaten erfolgt und der Zusatz von Stearaten zu Cholinchlorid-Pulvern mit organischem Trägermaterial keinen besonderen Einfluß auf die Fließfähigkeit besitzt.

Im erfindungsgemäßen Verfahren verwendet man als Trägermaterial Kieselsäure. Geeignete Kieselsäuren sind sogenannte Fällungskieselsäuren. Diese werden durch Reaktion einer wäßrigen Alkalisilicat-Lösung (z. B. Wasserglaslösung) mit Mineralsäuren (z. B. Schwefelsäure oder Salzsäure) mit sich anschließender Filtration und Sprüh- oder Wirbelschichttrocknung erhalten (vgl. dazu Ullmanns Encyklopädie der technischen Chemie, 4. Auflage, Band 21, S. 462f). Sie sollen eine Korngröße von 30 bis 250 µm aufweisen, wobei mehr als 50 Gew.%, insbesondere 60 Gew.% der Kieselsäuren eine Korngröße von 50 bis 160 µm aufweisen. Ihr Wassergehalt soll 4 bis 6 Gew.%, vorzugsweise 4,3 bis 6 Gew.%, betragen und ihre Stampfdichte bei Werten von 240 bis 300 g/l liegen.

Handelsübliche Fällungskieselsäuren, die diesen Kriterien genügen, sind z. B. Kieselsäuren mit dem

EP 0 158 120 B2

Namen Sipernat (R) (in der Bundesrepublik Deutschland eingetragenes Warenzeichen der Firma Degussa, Frankfurt).

Zum Abmischen verwendet man eine wäßrige Cholinchloridlösung, die 70 bis 80 Gew.%, vorzugsweise 77 bis 80 Gew.% und insbesondere 78 Gew.%, jeweils bezogen auf die Lösung, an Cholinchlorid enthält.

Wäßrige Cholinchloridlösung und Kieselsäure werden in solchen Gewichtsverhältnissen gemischt, daß man nach Beendigung des Mischvorgangs wasserhaltige Cholinchlorid-Kieselsäure-Pulver erhält, deren Gehalt an Cholinchiorid 40 bis 51 Gew.%, vorzugsweise 45 bis 51 Gew.% und insbesondere 50 bis 51 Gew% beträgt, jeweils bezogen auf die Mischung.

Prinzipiell ist es auch möglich, auf diese Weise zu Pulvern zu gelangen, deren Cholinchloridanteil kleiner als 40 Gew.% ist.

Vor dem Mischvorgang wird die wäßrige Cholinchloridlösung auf eine Temperatur von 40 bis 80 °C, vorzugsweise 50 bis 70 °C und insbesondere 60 °C erwärmt.

Der Mischvorgang wird in einer an sich bekannten Mischapparatur durchgeführt. Dabei legt man die Kieselsäure, deren Temperatur üblicherweise 20 bis 30 °C beträgt, vor und gibt dann in einem Zeitraum von 1 bis 4 Stunden die erwärmte wäßrige Cholinchloridlösung zu. Die Nachmischzeit beträgt im allgemeinen 10 bis 40 Minuten. Daran anschließend erfolgt die Zugabe von 0,05 bis 0,4 Gew.%, vorzugsweise 0,1 bis 0,2 Gew.%, jeweils bezogen auf die Mischung, von Magnesiumstearat oder Calciumstearat.

Die Stearate werden üblicherweise in Substanz, als fein zermahlene Pulver zugegeben. Es schließt sich ein weiterer Nachmischvorgang an, der ca. 5 bis 30 Minuten dauert.

Mittels der erfindungsgemäßen Verfahrens ist es auch möglich, getrocknete Cholinchlorid-Kieselsäure-Pulver mit einem Wassergehalt kleiner als 1,5 Gew.% und einem Cholinchloridgehalt von 40 bis 80 Gew.% vorzugsweise 51 bis 80 Gew.%, herzustellen.

Dazu werden wäßrige Cholinchloridlösung und Kieselsäure in entsprechenden Gewichtsverhältnissen gemischt, mit Magnesium- oder Calciumstearat versetzt und nach Beendigung des Mischvorgangs sprüh- oder wirbelschichtgetrocknet.

Eine andere Möglichkeit besteht darin, die wäßrige Cholinchloridlösung auf die Kieselsäure aufzusprühen, dieser Mischung Magnesium- oder Calciumstearat zuzusetzen und nach Beendigung des Mischvorgangs eine Trocknung bei einer Temperatur von 110 bis 150 °C vorzunehmen.

Die nach dem erfindungsgemäßen Verfahren hergestellten Cholinchlorid-Kieselsäure-Pulver zeichnen sich durch hervorragende Fließeigenschaften, gute Lagerstabilität und geringe Verbackungsneigung aus.

Zur Beurteilung der Fließfähigkeit werden Glasgefäße mit einem konischen Auslauf verwendet. Der Durchmesser der jeweiligen Auslauföffnungen liegt zwischen 0,5 cm und 5 cm. Die Fließfähigkeit wird folgendermaßen bestimmt: 100 g Cholinchlorid-Pulver muß frei und ohne Schütteln durch die Auslauföffnung fließen. Gelingt dieses bei einem Öffnungsdurchmesser von 0,5 cm, so wird das Fließverhalten als sehr gut bezeichnet. Bei einem Öffnungsdurchmesser von 0,6 bis 2 cm wird von einer guten Fließfähigkeit gesprochen, Bei einem Öffnungsdurchmesser von über 3 cm wird die Fließfähigkeit als nicht ausreichend angesehen.

Als lagerstabil werden Produkte bezeichnet, die, wenn sie über einen Zeitraum von 24 Stunden hinweg bei Raumtemperatur und einer Luftfeuchtigkeit von ca. 80 % gelagert wurden, noch eine gute Fließfähigkeit aufweisen.

Die folgenden Beispiele sollen die Erfindung näher erläutern.

Beispiel 1

2.75 kg Kieselsäure (Sipernat 22) mit einer Kornverteilung von 30 $\mu$m bis 195 $\mu$m und einem Wassergehalt von 4,7 Gew.% wurden in einem Mischer innerhalb von 2 Stunden mit 5 kg einer 77,87 gew.% igen wäßrigen Cholinchloridlösung, die auf 60 °C vorgewärmt war, versetzt. Dann wurde 20 Minuten nachgemischt. Die Fließfähigkeit in dieser Stufe war nicht ausreichend (Öffnungsdurchmesser: 4 cm).

Nach Zusatz von 9,75 g Calciumstearat und einer weiteren Mischungszeit von 8 Minuten war das Fließverhalten sehr gut (Öffnungsdurchmesser: 0,5 cm). Der Cholinchloridgehalt des Präparates lag bei 50,2 Gew.% und der Wassergehalt bei 22 Gew.%. Das Produkt ist lagerstabil. Es hatte nach 24-stündigem Stehen an der Luft (Luftfeuchtigkeit 80%) noch eine gute Fließfähigkeit (Öffnungsdurchmesser: 2 cm).

Beispiel 2

2,75 kg Kieselsäure (Sipernat 22) mit einer Kornverteilung von 30 $\mu$m bis 195 $\mu$m und einem Wassergehalt von 4,7 Gew.% wurden in einem Mischer innerhalb von 2 Stunden mit 5 kg einer 77,87 gew.% igen wäßrigen Cholinchloridlösung, die auf 60 °C vorgewärmt war, versetzt. Dann wurde 15 Minuten nachgemischt. Die Fließfähigkeit in dieser Stufe war nicht ausreichend (Öffnungsdurchmesser: 4 cm).

Nach Zusatz von 10,5 g Magnesiumstearat und einer weiteren Mischzeit von 8 Minuten war das Fließverhalten sehr gut (Öffnungsdurchmesser: 0,5 cm). Der Cholinchloridgehalt des Präparates lag bei 50,1 Gew.% und der Wassergehalt bei 21,9 Gew.%. Das Produkt ist lagerstabil. Es hatte nach 24-stündigem Stehen an der Luft (Luftfeuchtigkeit 75 %) noch eine gute Fließfähigkeit (Öffnungsdurchmesser: 2 cm).

Beispiele 3 und 4 (Vergleich)

Es wurde analog den Beispieien 1 und 2 verfahren, jedoch betrug die Temperatur der Cholinchloridlösung jeweils nur 25 °C. Nach dem Abmischen war die Fließfähigkeit beider Präparate nicht ausreichend (Öffnungsdurchmesser: > 3 cm).

Beispiel 5 (Vergleich)

2,75 kg Kieselsäure mit einer Kornverteilung von 20 μm bis 380 μm und einem Wassergehalt von 4 Gew.% wurden in einem Mischer innerhalb von 2 Stunden mit 5 kg einer 77,87 gew.% igen wäßrigen Cholinchloridlösung, die auf 60 °C vorgewärmt war, versetzt. Dann wurde 20 Minuten nachgemischt. Die Fließfähigkeit in dieser Stufe war nicht ausreichend (Öffnungsdurchmesser: > 5 cm). Das Produkt floß kaum.

Auch der Zusatz von 9,75 g Calciumstearat und eine weitere Mischungszeit von 10 Minuten konnten das Fließverhalten nicht verbessern.

**Patentansprüche**

1. Verfahren zur Herstellung von fließfähigen Cholinchlorid-Kieselsäure-Pulvern durch Mischen von Kieselsäure mit cholinchlorid, Zugeben von einem Erdalkalistearat und gegebenenfalls Trocknung dieser Mischung, dadurch gekennzeichnet, daß man sprüh- oder wirbelschichtgetrocknete Kieselsäure mit einer Korngröße von 30 bis 250 μm, die zu mehr als 50 Gew.-% eine Korngröße von 50 bis 160 μm aufweist, mit einer wäßrigen Cholinchloridlösung, die 70 bis 80 Gew.-%., bezogen auf die Lösung an Cholinchlorid enthält und deren Temperatur 40 bis 80°C beträgt, mischt und anschließend 0,05 bis 0,4 Gew.-%., bezogen auf die Mischung, an Magnesiumstearat oder Calciumstearat zugibt und diese Mischung gegebenenfalls trocknet.

2. Verfahren gemäß Anspruch 1, dadurch gekennzeichnet, daß man eine wäßrige Cholinchloridlösung verwendet, deren Temperatur 50 bis 70 °C beträgt.

3. Verfahren gemäß Anspruch 1, dadurch gekennzeichnet, daß man eine wäßrige Cholinchloridlösung verwendet, die 77 bis 80 Gew.% Cholinchlorid enthält.

4. Verfahren gemäß Anspruch 1, dadurch gekennzeichnet, daß man sprüh- oder wirbelschichtgetrocknete Kieselsäure mit einem Wassergehalt von 4 bis 6 Gew.% verwendet.

5. Verfahren gemäß Anspruch 1, dadurch gekennzeichnet, daß man 0,1 bis 0,2 Gew.% Magnesiumstearat oder Calciumstearat zugibt.

**Claims**

1. A process for the preparation of pourable choline chloride/silica powders by mixing silica with choline chloride, adding an alkaline earth metal stearate and, where appropriate, drying this mixture, wherein spray-dried or fluidized bed-dried silica of a particle size of from 30 to 250 μm, more than 50% by weight of which has a particle size of from 50 to 160 μm, is mixed with an aqueous choline chloride solution which contains from 70 to 80% by weight, based on the solution, of choline chloride and is at from 40 to 80°C, thereafter from 0.05 to 0.4% by weight, based on the mixture, of magnesium stearate or calcium stearate is added and, if appropriate, the resulting mixture is dried.

2. A process as claimed in claim 1, wherein an aqueous choline chloride solution at from 50 to 70°C is used.

3. A process as claimed in claim 1, wherein an aqueous choline chloride solution containing from 77 to 80% by weight of choline chloride is used.

4. A process as claimed in claim 1, wherein a spray-dried or fluidized bed-dried silica containing from 4 to 6% by weight of water is used.

5. A process as claimed in claim 1, wherein from 0.1 to 0.2% by weight of magnesium stearate or calcium stearate is added.

**EP 0 158 120 B2**

**Revendications**

1. Procédé de préparation de poudres fluides d'acide silicique et de chlorure de choline par mélange d'acide silicique et de chlorure de choline addition d'un stéarate alcalino-terreux et séchage éventuel de ce mélange, caractérisé en ce que l'on mélange de l'acide silicique séché par pulvérisation ou en lit fluidisé, d'une granulométrie comprise entre 30 et 250 um avec une proportion supérieure à 50 % en poids de particules aux dimensions comprises entre 50 et 160 um, et une solution aqueuse de chlorure de choline avec une teneur en chlorure de choline comprise entre 70 et 80 % de son poids, dont la température est maintenue entre 40 et 80 degrés C, puis on ajoute entre 0,05 et 0,4 % du poids du mélange de stéarate de magnésium ou de stéarate de calcium, ce mélange pouvant éventuellement être séché.

2. Procédé selon la revendication 1, caractérisé en ce que l'on emploie une solution aqueuse de chlorure de choline maintenue entre 50 et 70 degrés C.

3. Procédé suivant la revendication 1, caractérisé en ce que l'on emploie une solution aqueuse de chlorure de choline avec une teneur en chlorure de choline comprise entre 77 et 80 % en poids.

4. Procédé suivant la revendication 1, caractérisé en ce que l'on emploie de l'acide silicique séché par pulvérisation ou en lit fluidisé avec une teneur résiduelle en eau de 4 à 6 % en poids.

5. Procédé suivant la revendication 1, caractérisé en ce que l'on ajoute 0,1 à 0,2 % en poids de stéarate de magnésium ou de stéarate de calcium.